# EUROPEAN PATENT APPLICATION

(11) **EP 4 324 395 A1**
(43) Date of publication of application: **21.02.2024**
(21) Application number: 22788490.5
(22) Date of filing: 15.04.2022
(51) Int. Cl.: A61B 5/15, A61B 5/157, A61B 5/145, B01L 3/00

(54) **CASSETTE FOR BLOOD ANALYSIS**

(30) Priority: 16.04.2021 KR 20210050108
(71) Applicant: Careforu.co.,Ltd., Anyang city, Gyeonggi-do 13901 (KR)
(72) Inventor: KIM, Won Dong, Anyang-si Gyeonggi-do 14076 (KR)
(74) Representative: Grünecker Patent- und Rechtsanwälte PartG mbB
(86) International application number: PCT/KR2022/005449
(87) International publication number: WO 2022/220635

(57) **Abstract**

A CASSETTE FOR MEASURING A REACTION BETWEEN A SPECIMEN AND A SOLUTION, ACCORDING TO AN EMBODIMENT OF THE PRESENT APPLICATION, COMPRISES: A CARTRIDGE INCLUDING A FIRST CONTAINER AND A SECOND CONTAINER; AND A MAIN BODY INTO WHICH THE FIRST CONTAINER AND THE SECOND CONTAINER CAN BE INSERTED, WHEREIN THE MAIN BODY COMPRISES: A FIRST PLATE AND A SECOND PLATE WHICH ARE DISPOSED IN THE X-AXIS DIRECTION AND ARE LOCATED AT DIFFERENT POSITIONS IN THE Z-AXIS; A THIRD PLATE AND A FOURTH PLATE WHICH ARE DISPOSED IN THE Z-AXIS DIRECTION AND ARE LOCATED AT DIFFERENT POSITIONS ON THE X-AXIS; A FIFTH PLATE WHICH IS DISPOSED BETWEEN AT LEAST A PORTION OF THE SECOND PLATE AND THE FIRST PLATE IN THE X-AXIS DIRECTION; A BLOCKING WALL WHICH IS DISPOSED BETWEEN THE THIRD PLATE AND THE FOURTH PLATE IN THE Z-AXIS DIRECTION AND IS DISPOSED BETWEEN THE FIRST PLATE AND THE FIFTH PLATE WITH RESPECT TO THE Z-AXIS; AND A PARTITION WALL WHICH IS DISPOSED BETWEEN THE FIRST PLATE AND THE FIFTH PLATE WITH RESPECT TO THE Z-AXIS, BETWEEN THE THIRD PLATE AND THE BLOCKING WALL.

## Description

### [Technical Field]

The present invention relates to a cassette for blood analysis, and more particularly, to a cassette for measuring a reaction between a specimen and a solution, which minimizes a user's manual and improves reliability of test results of the specimen.

### [Background Art]

Recently, point-of-care testing performed to detect a target of biological specimens is widely used in the field of in vitro diagnostics. Point-of-care testing is advantageous in that it is low in cost and is capable of rapidly confirming diagnosis results, compared to a diagnostic method through general clinicopathological protocols which are performed in hospitals, bio-laboratories, and the like.

However, point-of-care testing is a diagnostic method performed according to a manual which is focused on user convenience without intervention of clinical judgement of medical persons, and has low reliability compared to the conventional diagnostic method. Therefore, there is growing demand for technology on a point-of-care testing method having improved reliability of diagnosis results.

### [Disclosure]

### [Technical Problem]

Therefore, the present invention has been made in view of the above problems, and it is an object of the present invention to provide a cassette having improved reliability for measuring a reaction between a specimen and a solution.

Objects to be solved by the present invention are not limited to the above-described objects, and other objects which have not been described above will be apparent to those skilled in the art from the specification and the accompanying drawings.

### [Technical Solution]

In accordance with the present invention, the above and other objects can be accomplished by the provision of a cassette for measuring a reaction between a specimen and a solution, having an x-axis, a y-axis, and a z-axis configured to be orthogonal to one another, and including a cartridge including a first container and a second container, and a main body configured such that the first container and the second container may be inserted thereinto, wherein the main body includes a first plate and a second plate disposed in the x-axis direction and located at different positions on the z-axis, a third plate and a fourth plate disposed in the z-axis direction and located at different positions on an x-axis, a fifth plate disposed in the x-axis direction between at least a part of the second plate and the first plate, a blocking wall disposed in the z-axis direction between the third plate and the fourth plate and disposed between the first plate and the fifth plate with respect to the z-axis, and separation walls disposed between the first plate and the fifth plate with respect to the z-axis, between the third plate and the blocking wall, a first accommodation space is defined by the first plate, the second plate, the third plate and the separation walls, a second accommodation space is defined by the first plate, the fifth plate, the blocking wall and the separation walls, a measurement space is defined by the first plate, the second plate, the fourth plate, the fifth plate and the blocking wall, a movement space is defined by the second plate and the fifth plate, the movement space is fluidly connected to the first accommodation space and the measurement space, the first accommodation space and the second accommodation space are fluidly connected to each other so that, when the main body is rotated in a first direction about the z-axis, a solution received in the second accommodation space is moved to the first accommodation space from the second accommodation space by gravity - a first guide configured to be located in the second accommodation space -, and the first accommodation space and the measurement space are fluidly connected to each other via the movement space so that, when the main body is rotated in a second direction opposite to the first direction about the z-axis, a solution received in the first accommodation space is moved to the measurement space from the first accommodation space by gravity.

Technical solutions of the present invention are not limited to the above-described technical solutions, and other technical solutions which have not been described above will become apparent to those skilled in the art from the specification and the accompanying drawings.

### [Advantageous effects]

According to one embodiment of the present invention, a first solution and a second solution are mixed with a specimen and/or react with the specimen, and are then moved to the measurement space via the movement space, provided separately, so as to be more smoothly moved to the measurement space, and the first guide, a second guide, and a third guide may be formed among the space configured to receive the first solution, the space configured to receive the second solution, and the movement space so that the first solution, the second solution, and the specimen may be effectively mixed and/or react with each other in the main body.

Further, the cassette according to the present invention may be produced in one process, compared to the conventional cassette produced by coupling a plurality of elements, and may thus increase convenience in production, and may not mix the first and second solutions received in the cassette without user action and operation of a device, and my thus improve reliability of diagnostic results.

Effects of the present invention are not limited to the above-described technical effects, and other effects which have not been described above will become apparent to those skilled in the art from the specification and the accompanying drawings.

### [Description of Drawings]

FIG. 1 is a flowchart representing one example of a general diagnosis protocol.
FIG. 2 is a block diagram illustrating a system for measuring a reaction between a specimen and a solution according to one embodiment.
FIG. 3 is a block diagram showing a cassette for measuring the rection between the specimen and the solution according to one embodiment.
FIG. 4 illustrates relations between a cartridge and a main body according to one embodiment.
FIG. 5 illustrates the cartridge according to one embodiment.
FIG. 6 is a view illustrating the external appearance of the main body according to one embodiment.
FIG. 7 is a top view of the main body according to one embodiment.
FIG. 8 is a cross-sectional view taken along line A-A' of FIG. 6.
FIG. 9 is a cross-sectional view taken along line B-B' of FIG. 6.
FIGs. 10 to 13 are views showing one example of a process of rotating the cassette according to one embodiment.

### [Mode for Invention]

The above and other objects, features and other advantages of the present invention will be more clearly understood from the following detailed description taken in conjunction with the accompanying drawings. However, the present invention will be described in conjunction with exemplary embodiments, and the present invention is intended to cover not only the exemplary embodiments, but also various alternatives, modifications, equivalents and other embodiments, which may be included within the spirit and scope of the invention as defined by the appended claims, and, hereinafter, reference will be made in detail to specific embodiments of the present invention, examples of which are illustrated in the accompanying drawings.

Wherever possible, the same reference numbers designate the same or like elements throughout the specification. Further, elements having the same functions within the same range of the sprit in the drawings in the respective embodiments are denoted by the same reference numerals, and a redundant description thereof will be omitted.

In the following description of the embodiments of the present invention, a detailed description of known functions and configurations incorporated herein will be omitted when it may make the subject matter of the present invention rather unclear. Further, in the following description of the embodiments, terms expressing ordinal numbers (for example, first and second) are used only to distinguish one element from other elements.

Further, in the following description of the embodiments, suffixes, such as "module" and "part", are provided or used interchangeably merely in consideration of ease in statement of the specification, and do not have meanings or functions distinguished from one another.

In the following description of the embodiments, singular expressions may be intended to include plural expressions as well, unless the context clearly indicates otherwise.

In the following description of the embodiments, the terms "comprising" and "having" are inclusive and therefore specify the presence of stated features and/or elements, but do not preclude the presence or addition of one or more other features and/or elements.

In the drawings, sizes of elements may be exaggerated or reduced for convenience of description. For example, the sizes and thicknesses of the respective elements in the drawings are expressed arbitrarily for convenience of description, and the present invention is not limited to the drawings.

When one embodiment may be implemented differently, a specific order of processes may not follow the order described herein. For example, two processes described consecutively may be substantially simultaneously performed, or may be performed in the opposite order to the described order.

In the following description of the embodiments, when an element or layer is referred to as being connected to another element or layer, it may be directly connected to the other element or layer, or may be indirectly connected to the other element or layer by intervening elements or layers present therebetween.

For example, when an element or layer is referred to as being electrically connected to another element or layer, it may be directly electrically connected to the other element or layer, or may be indirectly electrically connected to the other element or layer by intervening elements or layers present therebetween.

A cassette for measuring a reaction between a specimen and a solution according to one embodiment of the present invention has an x-axis, a y-axis, and a z-axis configured to be orthogonal to one another, and includes a cartridge including a first container and a second container, and a main body configured such that the first container and the second container may be inserted thereinto, the main body includes a first plate and a second plate disposed in the x-axis direction and located at different positions on the z-axis, a third plate and a fourth plate disposed in the z-axis direction and located at different positions on the x-axis, a fifth plate disposed in the x-axis direction between at least a part of the second plate and the first plate, a blocking wall disposed in the z-axis direction between the third plate and the fourth plate and disposed between the first plate and the fifth plate with respect to the z-axis, and separation walls disposed between the first plate and the fifth plate with respect to the z-axis, between the third plate and the blocking wall, a first accommodation space is defined by the first plate, the second plate, the third plate and the separation walls, a second accommodation space is defined by the first plate, the fifth plate, the blocking wall and the separation walls, a measurement space is defined by the first plate, the second plate, the fourth plate, the fifth plate and the blocking wall, a movement space is defined by the second plate and the fifth plate, the movement space is fluidly connected to the first accommodation space and the measurement space, the first accommodation space and the second accommodation space are fluidly connected to each other so that, when the main body is rotated in a first direction about the z-axis, a solution received in the second accommodation space is moved to the first accommodation space from the second accommodation space by gravity - a first guide configured to be located in the second accommodation space -, the first accommodation space and the measurement space are fluidly connected to each other via the movement space so that, when the main body is rotated in a second direction opposite to the first direction about the z-axis, a solution received in the first accommodation space is moved to the measurement space from the first accommodation space by gravity.

The separation walls may include a first separation wall and a second separation wall, the first separation wall may be connected to the first plate and be disposed in the z-axis direction between the third plate and the blocking wall, and the second separation wall may be connected to the first separation wall and the fifth plate.

The first container may receive a first solution, the second container may receive a second solution, and, when at least a part of the cartridge is inserted into the main body, at least a part of the first container and the first solution may be received in the first accommodation space, and at least a part of the second container and the second solution may be received in the second accommodation space.

The cartridge may include a specimen collector configured to hold the specimen, and, when at least the part of the cartridge is inserted into the main body, the specimen and the first solution may be mixed in the first accommodation space.

When the main body is rotated in the second direction about the z-axis, the first solution mixed with the specimen received in the first accommodation space is moved to the measurement space from the first accommodation space via the movement space by gravity.

When the main body is rotated in the first direction about the z-axis, at least a part of the first solution mixed with the specimen received in the measurement space is moved to the first accommodation space from the measurement space by gravity, and the second solution received in the second accommodation space is moved to the first accommodation space by gravity.

When the main body is rotated in the second direction about the z-axis, at least a part of the first solution mixed with the specimen and the second solution received in the first accommodation space may be moved to the measurement space from the first accommodation space via the movement space by gravity.

When the main body is rotated in the first direction about the z-axis, the solution received in the second accommodation space may be moved to the first accommodation space from the second accommodation space through the first guide, and at least a part of the first guide may be disposed between the separation walls and the blocking wall, and may have an upwardly inclined shape from one area of a bottom surface of the second accommodation space to the separation walls.

When the main body is rotated in the second direction about the z-axis, the solution received in the first accommodation space may be moved to the measurement space from the first accommodation space through a second guide configured to form at least a part of a bottom surface of the movement space.

The second guide may include a first detailed guide and a second detailed guide, the first detailed guide may be disposed between the first accommodation space and the second detailed guide, the second detailed guide may be disposed between the first detailed guide and the measurement space, the first detailed guide may be formed in an upwardly inclined shape from one end thereof connected to the first accommodation space to a remaining end thereof connected to the second detailed guide, and the second detailed guide may be formed in a downwardly inclined shape from one end thereof connected to the first detailed guide to a remaining end thereof.

A first area of the fifth plate located between the blocking wall and the fourth plate with respect to the x-axis may forms at least a part of one side surface of the measurement space, and a hall configured such that the solution may pass therethrough may be formed at a lower end of the first area.

A bottom surface of the first accommodation space and a bottom surface of the second accommodation space may not come into contact with each other, and may be physically separated from each other.

The second plate may include a first plane, a second plane, a third plane, a fourth plane, and a fifth plane, the first plane, the third plane, and the fifth plane may be disposed in the x-axis direction, a distance from the first plate to the first plane and the fifth plane in the z-axis direction may be less than a distance from the first plate to the third plane in the z-axis direction, the second plane may come into contact with the first plane and the third plane, and the fourth plane may come into contact with the third plane and the fifth plane.

The cartridge may further include a first container cover configured to seal the first container, a second container cover configured to seal the second container, and a holder, a position of the holder with respect to the cartridge may be changed due to contact with the second plate or the fifth plate, when at least the part of the cartridge is inserted into the main body, and, at least a part of the first container cover and at least a part of the second container cover may be respectively separated from the first container and the second container by the holder, when the position of the holder is changed.

Hereinafter, the cassette according to the present invention will be described with reference to the accompanying drawings.

### 1. Diagnosis Process

### 1.1 General Diagnosis Process

A diagnosis process indicates a set of protocols of acquiring information about a specific bio-target contained in a biological specimen which is to be diagnosed.

Although the diagnosis process has a detailed execution procedure varying depending on characteristics of the specific bio-target which is a target to be diagnosed, a general diagnosis process is executed through a process of finally acquiring diagnosis results by performing pre-treatment and post-treatment for detecting the specific bio-target.

In Enzyme-Linked ImmunoSorbent Assy (ELISA) protocols, which are the most representative method used in hospitals or laboratories, as the above-descried diagnosis process, a long time may be taken to derive diagnosis results due to a long time required for pre-treatment and post-treatment, and thereby, there are needs to acquire biological information by immediately deriving diagnosis results in an in vitro diagnostics market. In response to these needs in the market, among diagnosis processes for bio-targets to be diagnosed, point-of-care testing executed based on protocols which require a short processing time and are relatively simply implemented, is widely used.

### 1.2 Point-of-Care Testing

### 1-2-1 Definition

Point-of-care testing (POCT) is one of diagnosis methods in the field of in vitro diagnostics, and indicates a diagnostic technique which may reduce a processing time of a target of a biological sample without requiring medical person's additional clinical judgment so as to rapidly provide diagnosis results.

All kinds of samples, which may be taken from test objects (for example, people, animals, etc.), may be used as biological samples which are targets subjected to point-of-care testing, and a kind of sample which includes more kinds of targets for testing (for example, a specific protein, a specific lipid, a specific DNA, etc.) may be selected and determined as a target sample.

Further, in order to preform point-of-care testing, a kit in which a diagnosis protocol to detect whether or not a specific disease or a specific target (for example, a specific protein, a specific lipid, a specific DNA, or the like) is present or to measure the amount of the specific target is implemented is required.

Among diagnosis protocols, there is a diagnosis protocol using a method in which a reagent specifically binding to a specific protein is used to specifically bind to the specific protein in a sample, and then, remainders other than the protein specifically binding to the reagent are removed from a reaction result between the sample and the reagent in order to more accurately measure the amount of the protein specifically binding to the reagent.

In this case, when a diagnostic kit for point-of-care testing using the above-described diagnosis protocol is manufactured, the diagnostic kit should be designed such that the reaction result between the reagent and the sample may be separated into the protein specifically binding to the reagent and the remainders, and, in one of methods of designing such a diagnostic kit, the diagnostic kit is configured such that the reagent is combined with beads having a designated size or more so as to react with the sample, and then, the reaction result between the sample and the reagent is filtered by a filter or an absorption pad having a specific pore size using physical sizes.

The kit disclosed in the present invention is a diagnostic kit for point-of-care testing based on the above-described diagnosis protocol.

For example, one exemplary protocol implemented by point-of-care testing may include connecting a specific binder to a bio-target included in a specimen, and physically separating the bio-target to which the specific binding agent is connected. In the case of the protocol including physically separating the bio-target, an additional reaction procedure executed to measure information about the bio-target may be omitted, and thus, a time taken to output diagnosis results may be shortened. Therefore, recently, protocols including physically separating bio-targets are implemented in many cassettes for point-of-care testing. The diagnosis protocol for point-of-care testing including physically separating the bio-target will be described below.

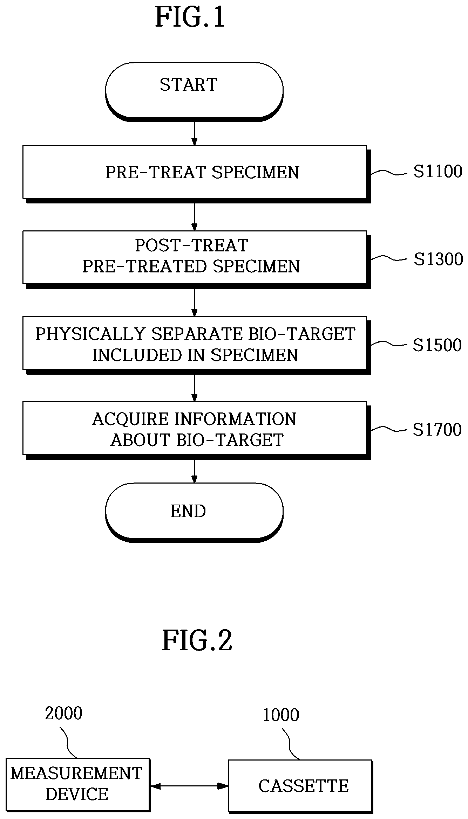

FIG. 1 is a flowchart representing one example of the general diagnosis protocol.

Referring to FIG. 1, the diagnosis protocol for point-of-care testing including physically separating the bio-target may include pre-treating a prepared specimen (S1100), post-treating the pre-treated specimen (S1300), physically separating the bio-target included in the specimen (S1500), and acquiring information about the bio-target (S1700).

First, the specimen including the bio-target may be provided. The specimen may be varied depending on the kind of the bio-target to be measured. For example, the specimen may be blood, urine, bodily fluid, or the like. When a specific target is determined, which sample is more suitable to detect the target may be determined/ Further, the target may be one selected from among various targets depending on a disease to be diagnosed or a kind of target to be detected. Particularly, the target may be a protein in blood cells or a protein in blood, including glycated hemoglobin or glycated albumin, and, when the target is a protein in blood cells or a protein in blood, including glycated hemoglobin or glycated albumin, the target specimen may be blood.

When the specimen is prepared, the specimen may be pre-treated (S1100). In this operation, "pre-treatment" means preliminary treatment of the prepared specimen through a chemical and/or physical action so that the information of the bio-target, which is a measurement target, may be measured from the outside. This may mean put the specimen in a state in which the bio-target is capable of specifically binding to a specific binder, which will be described below.

For example, glycated hemoglobin is a form of hemoglobin present in red blood cells that is chemically linked to a sugar and, because glycated hemoglobin is present in blood cells (red blood cells), in order to cause a `reaction' between the specific binder and glycated hemoglobin (more concretely, a reaction between a functional group regarding a sugar and the specific binder), it is necessary to destroy (remove) cell membranes of the red blood cells. In this case, a lysis solution for removing cell membranes reacts with the specimen, and thereby, glycated hemoglobin may be provided to the outside of the red blood cells so as to come into physical contact with foreign matter. Such a process is included in the 'pre-treatment' process.

When the pre-treatment has been completed, the pre-treated specimen may be post-treated (S1300). In this operation, a solution including at least the specific binder and/or an identifier linked to the specific binder or provided separately from the specific binder may be provided to the pre-treated specimen. Accordingly, the specimen and the solution may be mixed.

The specific binder is a substance which specifically binds to the bio-target so as to acquire the information about the bio-target by identifying or distinguishing the bio-target. When the bio-target is glycated hemoglobin, the specific binder may be boronic acid.

One example of the protocol for point-of-care testing may include physically separating the bio-target, as described above. That is, after the post-treatment has been performed, the bio-target included in the specimen may be physically separated (S1500). In the separation, the bio-target may be separated from other substances based on physical characteristics related to the specific binder binding to the bio-target in the post-treatment.

For example, when the specific binder is combined with beads having a designated size and the specific binder binds to the bio-target, the bio-target binding to the specific binder may be interpreted as having a particle size equal to or greater than the beads, and, when an absorption pad having pores having a size less than the beads is located under the solution including the bio-target in the direction of gravity, the binding bio-target is not moved into the absorption pad due to gravity and other substances having sizes less than the pores are moved (or absorbed) into the absorption pad, and consequently, the bio-target may be physically separated from the other substances.

Here, as one example, the beads may have a designated size. Concretely, the diameter of the beads may be 50 micrometers to 150 micrometers.

After the separation, the information about the bio-target may be acquired (S1700). Here, the information about the bio-target may mean whether or not the bio-target is present or the amount of the bio-target.

The information about the bio-target may be acquired by various methods. Among the methods of acquiring the information about the bio-target, there may be a method based on absorbance of the bio-target with respect to light in a specific wavelength band.

For example, when light in a wavelength band specifically absorbed by hemoglobin is radiated to a solution including hemoglobin and the intensity of reflected light is measured, information about the amount of hemoglobin included in the solution moved to an area, to which light is radiated, may be acquired. Here, the wavelength band of light specifically absorbed by hemoglobin may be 415 nanometers or a wavelength band in a range including at least 415 nanometers.

Hereinafter, a cassette for measuring a reaction between a specimen and a solution according to one embodiment will be described with reference to the accompanying drawings.

### 2. Cassette for Measuring Blood Sugar Information

### 2.1 Structure and Characteristics of Cassette

FIG. 2 is a block diagram illustrating a system for measuring a reaction between a specimen and a solution according to one embodiment. Referring to FIG. 2, the system may include a cassette 1000 and a measurement device 2000.

According to one embodiment, the reaction between the specimen and the solution may be measured using the cassette 1000 and the measurement device 2000.

The reaction between the specimen and the solution may be carried out by external action. More concretely, the cassette 1000 may receive a blood sample and the solution, and mixing between the blood sample and the solution received in the cassette 1000 and/or the reaction therebetween may be carried out by external action. As one example, mixing between the blood sample and the solution received in the cassette 1000 and/or the reaction therebetween may be carried out by user's action applied to the cassette 1000. As another example, mixing between the blood sample and the solution received in the cassette 1000 and/or the reaction therebetween may be carried out by operation of the measurement device 2000 applied to the cassette 100.

The mixed specimen and solution may be measured by the measurement device 2000. Concretely, the cassette 1000 may be coupled to the measurement device 2000, and the measurement device 2000 may measure the specimen and the solution received in the cassette 1000 coupled to the measurement device 2000 using various measurement methods.

Here, the measurement device 2000 may be a measurer configured to analyze or measure substances in the cassette 1000 coupled to the measurement device 2000. For example, the measurement device 2000 may include any one of various kinds of known devices which may perform analysis in the cassette 1000 coupled to the measurement device 2000.

Further, the cassette 1000 according to one embodiment is a diagnostic kit configured to acquire blood sugar information. Here, the blood sugar information may be acquired by measuring information about glycated hemoglobin or glycated albumin. Glycated hemoglobin is one kind of hemoglobin in which some functional groups of a sugar are linked to hemoglobin, and may be referred to as HbAlc. Glycated albumin is a protein included in red blood cells in blood, and indicated albumin to which glucose is linked.

Here, blood sugar information may mean information about the glycation rate of a substance to which glucose is linked (glycated hemoglobin or glycated albumin in one embodiment) other than the absolute amount of the substance. Therefore, in one embodiment, the ratio of the amount of glycated hemoglobin to the total amount of hemoglobin (or the amount of glycated albumin to the total amount of albumin) may be measured to acquire the blood sugar information.

Further, the cassette according to one embodiment may be a kit for measuring various factors other than glycated hemoglobin or glycated albumin. For example, the cassette according to one embodiment may be a diagnostic kit for measuring an albumin-to-creatinine ratio (ACR), C-reactive protein (CRP), lysophosphatidylcholine (LPC), total cholesterol (TC), or the like.

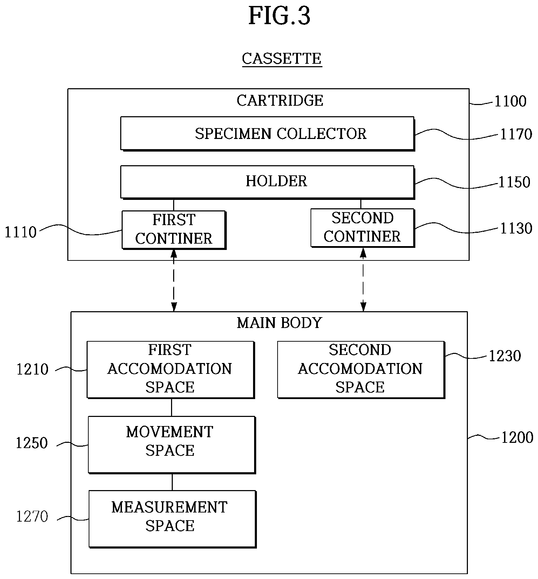

FIG. 3 is a block diagram showing the cassette according to one embodiment. Referring to FIG. 3, the cassette according to one embodiment may include a cartridge 1100 and a main body 1200.

The cartridge 1100 may include a first container 1110, a second container 1130, a holder 1150, and a specimen collector 1170. Further, the main body 1200 may include a first accommodation space 1210, a second accommodation space 1230, a movement space 1250, and a measurement space 1270.

The holder 1150 may be directly or indirectly connected to at least a part of the first container 1110 and at least a part of the second container 1130. Concretely, the holder 1150 may perform a function of leaking solutions received in the first container 1110 and the second container 1130 to the outside, as the cartridge 1100 is coupled to the main body 1200.

At least a part of the cartridge 1100 may be inserted into the main body 120 so as to perform mixing between the specimen held by the specimen collector 1170 and the solution and/or the reaction therebetween. Here, the first accommodation space 1110 may receive the first container 1110, and the second accommodation space 1130 may receive the second container 1130, depending on coupling between the cartridge 1100 and the main body 1200.

Hereinafter, the respective elements included in the cartridge and the main body and coupling relations between the cartridge and the main body will be described in more detail.

### 2.2.1 Coupling Relations between Cartridge and Main Body

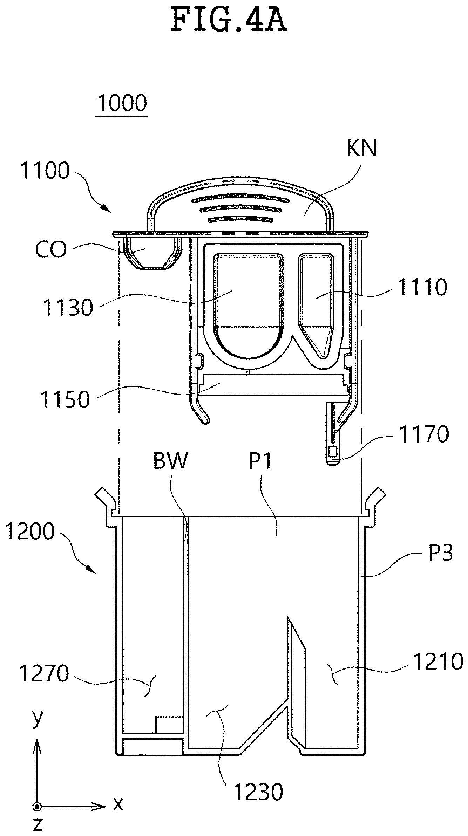

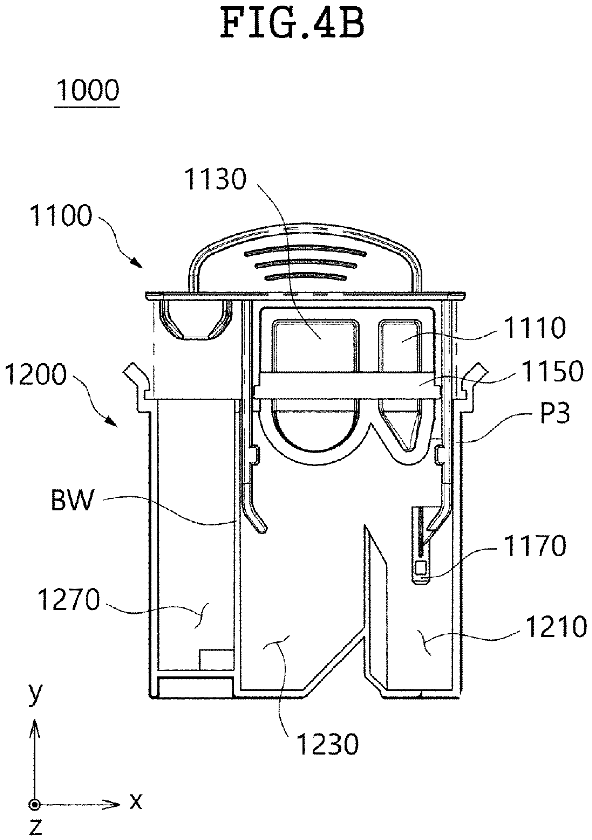

FIG. 4 illustrates the relations between the cartridge and the main body according to one embodiment.

Referring to FIG. 4, (a) is a view showing a state in which the cartridge 1100 and the main body 1200 are separated from each other, and (b) is a view showing a state in which the cartridge 1100 is inserted into the main body 1200.

When mixing between the specimen and the solution and/or the reaction therebetween is not carried out in the cassette 1100 according to one embodiment, the cartridge 1100 and the main body 1200 may be separated from each other, as shown in FIG. 4(a).

Further, in order to perform mixing between the specimen and the solution and/or the reaction therebetween, at least a part of the cartridge 1100 of the cassette 1000 according to one embodiment may be inserted into the main body 1200, as shown in FIG. 4(b).

When at least a part of the cartridge 1100 is inserted into the main body 1200, the first container 1110 or the second container 1130 may be received in the main body 1200, as one example. In this case, the first container 1110 and the second container 1130 may be disposed in the upper ends of the first accommodation space 1210 and the second accommodation space 1230, respectively. Thereby, when the solutions received in the first container 1110 and the second container 1130 leak from the first container 1110 and the second container 1130, the solutions leaked from the first container 1110 and the second container 1130 may be moved to the first accommodation space 1210 and the second accommodation space 1230.

Of course, even in the case that at least a part of the first container 1110 is received in the first accommodation space 1210 and at least a part of the second container 1130 is received in the second accommodation space 1230, when the solutions received in the first container 1110 and the second container 1130 leak from the first container 1110 and the second container 1130, the solutions leaked from the first container 1110 and the second container 1130 may be moved to the first accommodation space 1210 and the second accommodation space 1230, respectively. A more detailed description thereof will be given below.

### 2.1.2 Configuration and Shape of Cartridge

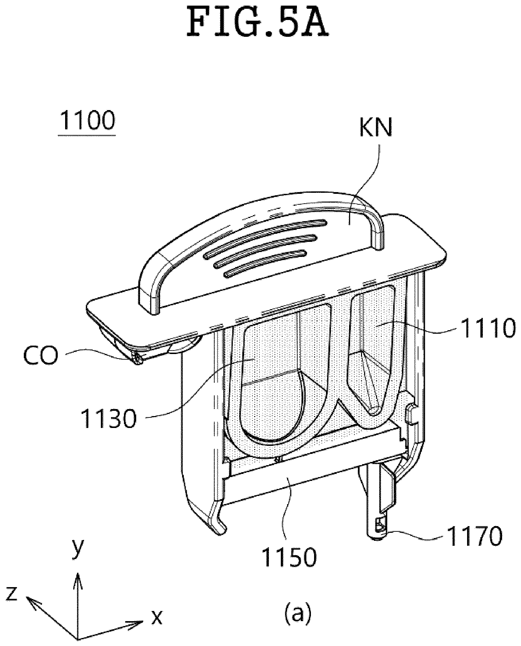

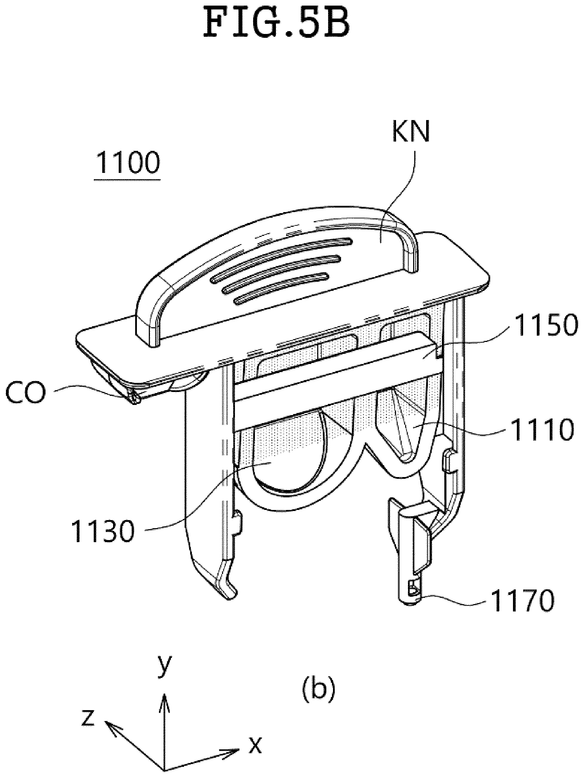

FIG. 5 illustrates the cartridge according to one embodiment.

Referring to FIG. 5, the cartridge 1100 may include the first container 1110, the second container 1130, the holder 1150, and the specimen collector 1170.

The first container 1110 and the second container 1130 may have inner spaces configured to receive solutions. For example, the first container 1110 may have an inner space configured to receive a first solution, and the second container 1130 may have an inner space configured to receive a second solution. In this case, the first solution and the second solution may be the same solution or different solutions.

Concretely, the first container 110 may receive a solution including a substance which performs a function of pre-treating the specimen and a substance which may specifically bind to the bio-target included in the specimen. For reference, in the binding relations between the bio-target and the substance which may specifically bind to the bio-target, specific binding may be formed at a functional group of the bio-target indicating glucose. That is, when the bio-target is glycated hemoglobin, the part of the bio-target forming binding relations with the substance which may specifically bind to the bio-target is a functional group of glycated hemoglobin indicating glucose, and, when the bio-target is glycated albumin, the part of the bio-target forming binding relations with the substance which may specifically bind to the bio-target may be a functional group of glycated albumin indicating glucose.

For example, the specimen may be a blood sample, and the substance which performs the function of pre-treating the specimen may be a substance configured to hemolyze blood cells of the blood sample. For example, the substance which performs the function of pre-treating the specimen may be an N-2-hydroxyethylpiperazine-N'-2-ethanesulfonic acid (HEPES; pH 8.1) buffering solution as a surfactant.

Further, the substance which may specifically bind to the bio-target may be a specific binder for the bio-target. The substance which may specifically bind to the bio-target (glycated hemoglobin or glycated albumin) may be representatively boronic acid, without being limited thereto.

According to one embodiment, the specific binder may be provided as being combined with beads. According to one embodiment, the beads may be polysaccharide support beads formed of polymethyl methacrylate (PMMA), agarose, cellulose or Sepharose, glass beads, or latex beads.

Hereinafter, the solution including the substance which performs the function of pre-treating the specimen and the substance which may specifically bind to the bio-target (that is, the functional group of glycated hemoglobin indicating glucose or the functional group of glycated albumin indicating glucose) included in the specimen will be referred to the first solution.

The first solution may be implemented in the form of a mixed solution acquired by mixing the substance which performs the function of pre-treating the specimen and the substance which may specifically bind to the bio-target.

Further, the first solution may include beads configured such that the substance which may specifically bind to the bio-target is combined therewith, in addition to the substance which performs the function of pre-treating the specimen. Concretely, the first solution may include beads coated with boronic acid and having a size of (50 micrometers to 150 micrometers).

The second container 1130 may receive a solution including a substance configured to promote movement of other substances other than the bio-target in the reaction result between the specimen and first solution to the absorption pad, which will be described below. For example, the substance configured to promote the movement of the other substances may be referred to as a buffer solution. Hereinafter, the solution received in the second container 1130 will be referred to as a washing solution. Further, the second solution may include a color former configured to measure and quantify light reflected by the reaction result of the first solution or absorbance of the reaction result. For example, the color former may be BCG, BCP, DA-67, 4-AAP, TOOS, ADOS, MAOS, thionine, or the like.

Further, the cartridge 1100 may include a first container cover and a second container cover configured to seal the first container 1110 and the second container 1130. The first container cover may seal the first container 1110, and may thus perform a function of preventing the solution received in the first container 1110 from leaking to the outside. Further, the second container cover may seal the second container 1130, and may thus perform a function of preventing the solution received in the second container 1130 from leaking to the outside.

Here, the first container cover and the second container cover may be provided as covers physically separated from each other, without being limited thereto, or may be provided as one physically integrated cover. For example, the first container cover and the second container cover may be provided as one screen provided to seal the first container 1110 and the second container 1130, as shown in FIG. 5(a) . In this case, the first container cover and the second container cover may be formed of aluminum foil, without being limited thereto, or may be formed of various materials which may perform a function of preventing the solutions received in the first container 1110 and the second container 1130 from leaking to the outside.

The cartridge 1110 may include the holder 1150 directly or indirectly connected to at least a part of the first container 1110 and at least a part of the second container 1130. The holder 1150 may be directly or indirectly connected to at least a part of the first container 1110 and at least a part of the second container 1130, and may perform a function of leaking the first solution received in the first container 1110 and the second solution received in the second container 1130 to the outside.

As one example, the holder 1150 may be directly connected to at least a part of the first container 1110 and at least a part of the second container 1130. In this case, the holder 1150 may be provided such that at least a part thereof comes into contact with the lower parts of the first container 1110 and the second container 1130.

As another example, the holder 1150 may be indirectly connected to at least a part of the first container 1110 and at least a part of the second container 1130. In this case, a separate connection member may be provided between the holder 1150, and the first container 1110 and the second container 1130. More concretely, the holder 1150 may be indirectly connected to at least a part of the first container 1110 and at least a part of the second container 1130 through the separate connection member.

According to one embodiment, at least a part of the holder 1150 may be connected to the first container cover sealing the first container 1110 and the second container cover sealing the second container 1130. In this case, the holder 1150 comes into contact with at least one of a second plate P2 or a fifth plate P5 of the main body 1200, which will be described below, and thereby, the holder 1150 may be separated from the cartridge 1100 and thus relative positions of the holder 1150 and the cartridge 1100 may be changed.

For example, when the cartridge 1100 is inserted into the main body 1200, the position of the holder 1150 with respect to the cartridge 1100 may be changed due to contact of the holder 1150 with at least one of the second plate P2 or the fifth plate P5. Thereby, at least a part of the first container cover may be separated from the first container 1110, and at least a part of the second container cover may be separated from the second container 1130. According to another embodiment, the holder 1150 may be physically separated from the first container cover sealing the first container 1110 and the second container cover sealing the second container 1130.

Here, when the cartridge 1100 is inserted into the main body 1200, the position of the holder 1150 with respect to the cartridge 1100 may be changed due to contact of the holder 1150 with at least one of the second plate P2 or the fifth plate P5. Thereby, the first container cover sealing the first container 1110 and the second container cover sealing the second container 1130 may be separated from the first container 1110 and the second container 1130, respectively. As one example, as the position of the holder 1150 with respect to the cartridge 1110 is changed, the holder 1150 may separate the first container cover and the second container cover from the first container 1110 and the second container, respectively. As another example, as the position of the holder 1150 with respect to the cartridge 1110 is changed, the holder 1150 may scratch at least the part of the first container cover and at least the part of the second container cover.

More concretely, FIG. 5(a) is a view showing the state of the holder before the position of the holder is changed, and FIG. 5(b) is a view showing the state of the holder when the position of the holder has been changed.

Before the position of the holder is changed, as shown in FIG. 5(a), the first container cover and the second container cover may respectively seal the first container 1110 and the second container 1130. That is, before the position of the holder is changed, leakage of the solution received in the first container 1110 and the solution received in the second container 1130 to the outside may be prevented by the first container cover and the second container cover.

Thereafter, as the position of the holder 1150 has been changed, as shown in FIG. 5(b), by inserting at least a part of the cartridge 1100 into the main body 1200, at least a part of the first container cover may be separated from the first container 1110, and at least a part of the second container cover may be separated from the second container 1130. Thereby, the first solution received in the first container 1110 and the second solution received in the second container 1130 may flow into the main body 1200.

For example, when at least a part of the cartridge 1100 is inserted into the main body 1200, the first solution may be received in the first accommodation space 1210. Further, the second solution may be received in the second accommodation space 1230.

The cartridge 1100 may include the specimen collector 1170 configured to receive the specimen. When the cartridge 1110 is not inserted into the main body 1200, at least a part of the specimen collector 1170 may be exposed to the outside so as to receive the specimen.

The specimen collector 1170 may have a through hole configured to hold a liquid specimen through a capillary phenomenon, when the liquid specimen is provided. For example, when a blood sample is provided to the specimen collector 1170, the specimen collector 1170 may hold the blood sample in the through hole.

The through hole of the specimen collector 1170 may be formed to face in a direction of inserting the cartridge 1100 into the main body 1200, for example, in the negative direction of the y-axis in FIG. 5. Further, the specimen collector 1170 may be located at a lower position than the above-described holder 1150 in the cartridge 1100. Thereby, when the cartridge 1100 is inserted into the main body 1200, the specimen collector 1170 may be inserted into the main body 1200 before the first container 1110, the second container 1130, and the holder 1150.

The cartridge 1110 may include a knob KN. The knob KN may be formed to grip the cartridge 1110, and may assist insertion of the cartridge 1110 into the main body 1200.

The cartridge 1110 may include a measurement area cover CO. The measurement area cover CO may be inserted into the upper part of the measurement space 1270 of the main body 1200, which will be described below, so as to prevent the solution received in the measurement space 1270 from leaking.

### 2.1.3 Configuration and Shape of Main Body

The main body 1200 according to one embodiment may have a plurality of spaces configured to receive solutions, and the plurality of spaces may be defined by a plurality of members forming the main body 1200, for example, a plurality of plates and a plurality of walls. Hereinafter, the plurality of members forming the main body 1200 and the plurality of spaces formed by the plurality of members will be descried with reference to the accompanying drawings.

### 2.1.3.1 Elements Forming Main Body

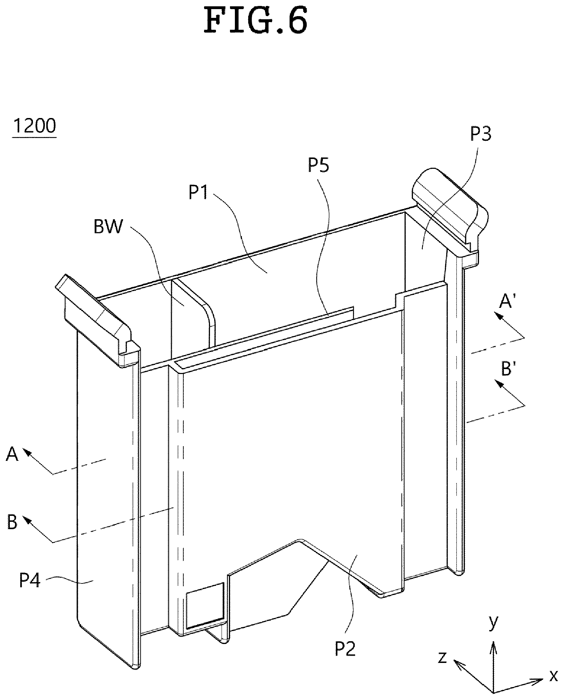

FIG. 6 is a view illustrating the external appearance of the main body according to one embodiment.

Referring to FIG. 6, the main body 1200 may include the plurality of plates. Here, the plates may be provided in various shapes other than a rectangular parallelepiped shape, and may be formed by one or more planes. For example, the second plate P2 may be formed by a plurality of planes having different shapes, sizes and placement positions.

The main body 1200 may include a first plate P1, the second plate P2, a third plate P3, a fourth plate P4, and the fifth plate P5. In this case, when a user looks at the first plate P1, the first plate P1 may correspond to the front plate of the main body 1200, the second plate P2 may correspond to the rear plate of the main body 1200, the third plate P3 may correspond to the left plate of the main body 1200, and the fifth plate P4 may correspond to the right plate of the main body 1200. The plurality of spaces formed in the main body 1200 may be defined by the above-described plurality of plates.

The first plate P1 and the second plate P2 may be disposed in the x-axis direction in FIG. 6. Otherwise, the first plate P1 and the second plate P2 may be disposed substantially perpendicular to the z-axis direction in FIG. 6. For example, the first plate P1 and the second plate P2 may be disposed parallel to each other in the x-axis direction. The second plate P2 may be disposed such that at least a part thereof is parallel to the first plate P1 in the x-axis direction. Otherwise, the second plate P2 may be disposed such that at least a part thereof is spaced apart from the first plate P1 in the z-axis direction.

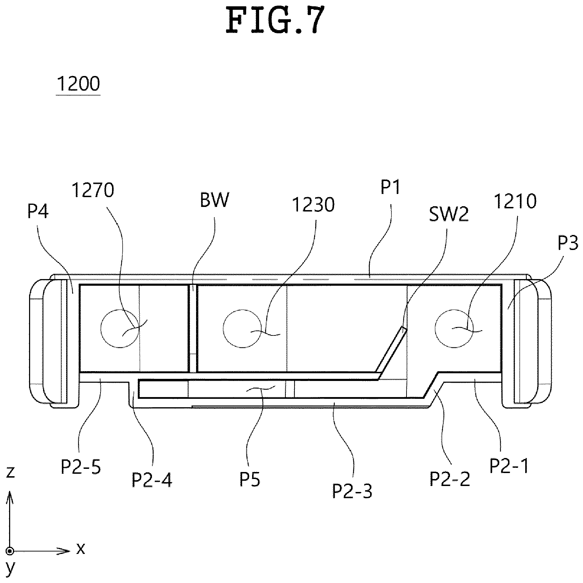

FIG. 7 is a top view of the main body according to one embodiment. Referring to FIG. 7, the second plate P2 may include detailed planes. The second plate P2 may include a first plane P2-1, a second plane P2-2, a third plate P2-3, a fourth plane P2-4, and a fifth plane P2-5.

The first plane P2-1, the third plane P2-4, and the fifth plane P2-5 may be disposed in the x-axis direction in FIG. 7, or may be disposed substantially perpendicular to the z-axis direction in FIG. 7. Here, the first plane P2-1, the third plane P2-4, and the fifth plane P2-5 may be disposed to be spaced apart from the first plate P1 in the z-axis direction, and a distance between the third plane P2-3 and the first plate P1 may be greater than a distance between the first plane P2-1 or the fifth plane P2-5 and the first plate P1. Further, the first plane P2-1 may come into contact with at least a part of the third plate P3 to be orthogonal thereto, and the fifth plane P2-5 may come into contact with at least of the fourth plate P4 to be orthogonal thereto.

The second plane P2-2 may come into contact with the first plane P2-1 and the third plane P2-3. More concretely, one side of the second plane P2-2 may come into contact with the first plane P2-1, and the other side of the second plane P2-2 may come into contact with the third plane P2-3. The fourth plane P2-4 may come into contact with the third plane P2-3 and the fifth plane P2-5. More concretely, one side of the fourth plane P2-4 may come into contact with the third plane P2-3, and the other side of the fourth plane P2-4 may come into contact with the fifth plane P2-5. At least a part of the second plate P2, for example, the third plane P2-3, may be spaced apart from the fifth plate P5 by the second plane P2-2 and the fourth plane P2-4.

The third plate P3 and the fourth plate P4 may be disposed in the z-axis direction in FIG. 6. Otherwise, the third plate P3 and the fourth plate P4 may be disposed substantially perpendicular to the x-axis direction in FIG. 6. As one example, the third plate P3 and the fourth plate P4 may be disposed parallel to each other in the z-axis direction. Otherwise, the third plate P3 may be disposed to be spaced apart from the fourth plate P4 by a designated distance in the x-axis direction. Here, the distance from the first pate P1 to at least the part of the second plate P2 spaced apart from the first plate P1 in the z-axis direction may be less than the distance from the fourth plate P4 to the third plate P3 spaced apart from the fourth plate P4 in the x-axis direction. That is to say, the distance between the third plate P3 and the fourth plate P4 may be greater than the distance between the first plate P1 and the second plate P2.

The fifth plate P5 may be disposed in the x-axis direction, and may be disposed between at least the part of the second plate P2 and the first plate P1.

The fifth plate P5 may be spaced apart from the first plate P1 by a first distance in the z-axis direction, and may be spaced apart from at least the part of the second plate P2 by a second distance, and the first distance may be greater than the second distance.

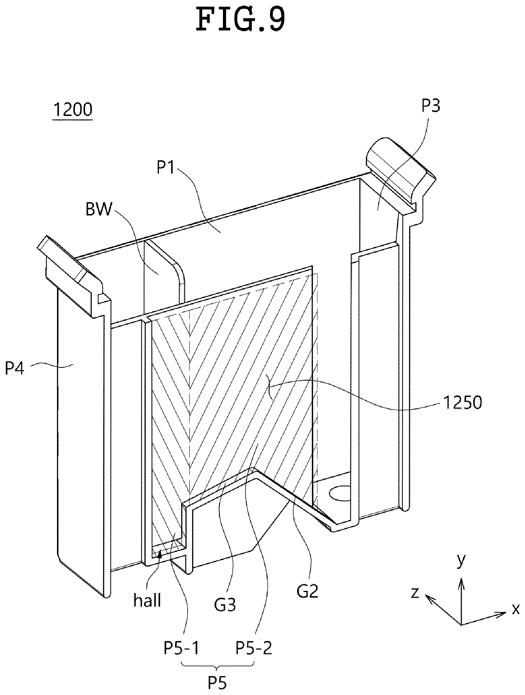

Referring to FIG. 9, the fifth plate P5 may include a first area P5-1 configured to form at least a part of one side surface of the measurement space 1270. Further, the fifth plate P5 may include a second area P5-2 configured to form at least a part of one side surface of the second accommodation space 1230. Here, a hall configured such that the solution may pass therethrough may be formed at the lower end of the first area P5-1. The solutions received in the first accommodation space 1210 (or the measurement space 1270) may be moved to the measurement space 1270 (or the first accommodation space 1210) via the hall by rotation of the main body 1200.

The main body 1200 may include a plurality of walls. Here, the plurality of spaces formed in the main body 1200 may be defined by the plurality of walls.

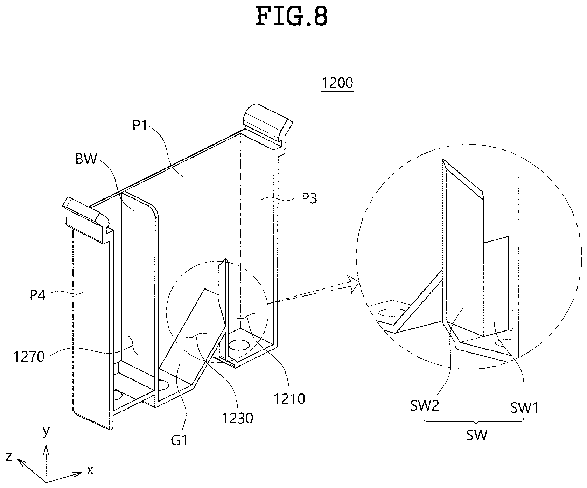

FIG. 8 is a cross-sectional view taken along line A-A' of FIG. 6, and FIG. 9 is a cross-sectional view taken along line B-B' of FIG. 6.

Referring to FIGs. 6 and 8, the main body 1200 may include separation walls SW and a blocking wall BW. The separation walls SW and the blocking wall BW may be disposed in the z-axis direction in FIG. 6. Otherwise, at least some of the separation walls SW and the blocking wall BW may be disposed substantially perpendicular to the x-axis direction in FIG. 6.

The separation walls SW may serve to separate the first accommodation space 1210 and the second accommodation space 1230 from each other. That is, the solution received in the first accommodation space 1210 and the solution received in the second accommodation space 1230 are not mixed due to the separation walls SW as long as no external force is applied to the main body 1200. On the other hand, when external force is applied to the main body 1200, the solution received in the second accommodation space 1230 may be moved to the first accommodation space 1210, and a detailed description thereof will be given below.

The blocking wall BW serves to separate the second accommodation space 1230 and the measurement space 1270 from each other. Concretely, the blocking wall BW may block spatial connection between the second accommodation space 1230 and the measurement space 1270. That is, the blocking wall BW functions to prevent the solution received in the second accommodation space 120 from being moved to the measurement space 1270. Thereby, even when external force is applied to the main body 1200, the solution received in the second accommodation space 1230 and the solution received in the measurement space 1270 are not mixed. /Here, the height of the blocking wall BW may be greater than the heights of the separation walls SW.

Referring to an enlarged view in FIG. 8, the separation walls SW may include a first separation wall SW1 and a second separation wall SW2. The first separation wall SW1 may be connected to the first plate P1, and may be disposed in the z-axis direction in FIG. 8 between the third plate P3 and the blocking wall BW. Further, the second separation wall SW2 may be formed to be connected to the first separation wall SW1 and the fifth plate P5.

More concretely, the first separation wall SW1 may be connected perpendicularly to the first plate P1. Further, the first separation wall SW1 may be disposed in the z-axis direction in FIG. 8 parallel to the third plate P3, the fourth plate P4 or the blocking wall BW. The second separation wall SW2 may be disposed so as not to be perpendicular to the x-axis and the y-axis in FIG. 8. The second separation wall SW2 is disposed at an angle different from the first separation wall SW1 on the x-z plane in FIG. 8, and thereby, the solution received in the first accommodation space 1210 may be more smoothly moved to the movement space 1250.

The second separation wall SW2 may be formed to have a greater height than the first separation wall SW1 based on the y-axis in FIG. 8. That is, the length of the second separation wall SW2 in the y-axis direction in FIG. 8 may be greater than the length of the first separation wall SW1 in the y-axis direction in FIG. 8.

As the second separation wall SW2 is formed to have the greater height than the first separation wall SW1 based on the y-axis in FIG. 8, the second separation wall SW2 may serve as a barrier configured to prevent the solution received in the first container 1110 from flowing into the second accommodation space 1230 when the cartridge 1100 is inserted into the main body 1200.

Further, as the second separation wall SW2 is formed to have the greater height than the first separation wall SW1 based on the y-axis in FIG. 8, the second separation wall SW2 may serve as a guide configured to assist at least a part of the solution received in the first accommodation space 1210 to be moved to the movement space 1250 depending on change in the position of the main body 1200. Moreover, as the second separation wall SW2 is formed to have the greater height than the first separation wall SW1 based on the y-axis in FIG. 8, the second separation wall SW2 may serve as a guide configured to assist at least a part of the solution received in the second accommodation space 1230 to be moved to the first accommodation space 1210.

The first separation wall SW1 and the blocking wall BW may be disposed parallel to each other in the z-axis direction. As another example, the separation walls SW and the blocking wall BW may be disposed to be spaced apart from each other in the x-axis direction.

The blocking wall BW may be disposed in the z-axis direction between the third plate P3 and the fourth plate P4, and may be disposed between the first plate P1 and the fifth plate P5 with respect to the z-axis. The separation walls SW may be disposed between the third plate and the blocking wall BW, and may be disposed between the first plate P1 and the fifth plate P5 with respect to the z-axis.

When the cartridge 1100 is inserted into the main body 1200, at least a part of the holder 1150 of the cartridge 1100 may come into contact with the second plate P2 or the fifth plate P5 of the main body 1200, and thereby, the position of the holder 1150 may be changed. As the position of the holder 1150 is changed, at least a part of the first container over and a part of the second container cover which come into contact with at least a part of the holder 110 may be separated from the first container 1110 and the second container 1130, respectively, and thereby, the solution received in the first container 1110 may flow to be received in the first accommodation space 1210, and the solution received in the second container 1130 may flow to be received in the second accommodation space 1230.

As described above, the plurality of spaces formed in the main body 1200 may be defined based on at least one of the plurality of plates (for example, the first plate P1 to the fifth plate P5) or the plurality of walls (for example, the separation walls SW and the blocking wall BW), and a detailed description thereof will be given below with reference to the accompanying drawings.

### 2.1.3.2 Spaces Defined by Elements forming Main Body

Referring to FIGs. 8 and 9, the main body 1200 may include the plurality of spaces. Concretely, the main body 1200 may include the first accommodation space 1210, the second accommodation space 1230, the movement space 1250, and the measurement space 1270. Here, the first accommodation space 1210, the second accommodation space 1230, the movement space 1250, and the measurement space 1270 may mean physically independent spaces, or may mean spaces which are not physically separated but are functionally separated. For example, the first accommodation space 1210 is fluidly connected to the movement space 1250, but, when external force is not applied to the main body 1200 and a designated amount or less of the solution is received in the first accommodation space 1210, the solution received in the first accommodation space 120 is not moved to the movement space 1250, and therefore, the first accommodation space 1210 and the movement space 1250 may be functionally separated. Further, although not shown in the drawings, the main body 1200 may include a bottom surface.

The first accommodation space 1210 may be defined by at least parts of the first plate P1, the second plate 2, the third plate P3, the separation walls SW and the bottom surface. Otherwise, the first accommodation space 1210 may be defined by at least a part of the first plate P1, at least a part of the second plate P2, the third plate P3, the separation walls SW, and at least a part of the bottom surface.

The second accommodation space 1230 may be defined by at least parts of the first plate P1, the fifth plate P5, the blocking wall BW, the separation walls SW and the bottom surface. Otherwise, the second accommodation space 1230 may be defined by at least a part of the first plate P1, at least a part of the fifth plate P5, the blocking wall BW, the separation walls SW, at least a part of the bottom surface, and a first guide G1.

The measurement space 1270 may be defined by at least parts of the first plate P1, the second plate P2, the fourth plate P4, the blocking wall BW and the bottom surface. Otherwise, the measurement space 1270 may be defined by at least parts of the first plate P1, the second plate P2, the fourth plate P4, the fifth plate P5, the blocking wall BW and the bottom surface. Otherwise, the measurement space 1270 may be defined by at least a part of the first plate P1, at least a part of the second plate P2, at least a part of the fourth plate P4, at least a part of the fifth plate P5, at least a part of the bottom surface, and the blocking wall BW.

The movement space 1250 may be defined by at least parts of the second plate P2, the fifth plate P5, and the bottom surface. More concretely, the movement space 1250 may be defined by at least parts of the plurality of detailed planes, forming the second plate P2, and the bottom surface. As one example, the movement space 1250 may be defined by at least parts of the second plane P2-2, the third plane P2-3, the fourth plane P2-4, the fifth plate P5 and the bottom surface. As another example, the movement space 1250 may be defined by at least parts of the second plane P2-2, the third plane P2-3, the fourth plane P2-4, the fifth plane P2-5, the fifth plate P5 and the bottom surface.

### 2.1.3.3 Functions of Respective Spaces

The first accommodation space 1210 may be provided as a space configured to receive a solution. One or more kinds of solutions may be received in the first accommodation space 1210. For example, the first accommodation space 1210 may receive the first solution, or may receive a mixed solution of the first solution and the second solution.

The first accommodation space 1210 may be provided as a space configured to receive the solution received in the first container 1110. That is to say, as at least a part of the cartridge 1100 is inserted into the main body 1200, at least a part of the solution received in the first container 1110 may be moved to the first accommodation space 1210. Concretely, when at least a part of the cartridge 1100 is inserted into the main body 1200, the solution in the first container 1110 may be received in the first accommodation space 1210 depending on change in the position of the holder 1150 due to contact of the holder 1150 of the cartridge 1100 with the second plate P2 or the fifth plate P5 forming the main body 1200. As this was described above, a detailed description thereof will thus be omitted because it is considered to be unnecessary.

Further, as at least a part of the cartridge 1100 is inserted into the main body 1200, a specimen rod may be located in the first accommodation space 1210. Thereby, mixing between a specimen and the first solution and/or a reaction therebetween may be performed in the first accommodation space 1210.

The second accommodation space 1230 may be provided as a space configured to receive a solution. The second accommodation space 1230 may be provided as a space configured to receive a different kind of solution from the solution received in the first accommodation space 1210.

The second accommodation space 1210 may be provided as a space configured to receive the solution in the second container 1130. That is to say, as at least a part of the cartridge 1100 is inserted into the main body 1200, at least a part of the solution in the second container 1130 may be moved to the second accommodation space 1230. Concretely, when at least a part of the cartridge 1100 is inserted into the main body 1200, the solution in the second container 1130 may be received in the second accommodation space 1230 depending on change in the position of the holder 1150 due to contact of the holder 1150 of the cartridge 1100 with the second plate P2 or the fifth plate P5 forming the main body 1200. As this was described above, a detailed description thereof will be omitted because it is considered to be unnecessary.

The movement space 1250 may be provided as a space configured to move the solution from the first accommodation space 1210 to the measurement space 1270 therethrough. As one example, at least a part of the solution received in the first accommodation space 1210 may be moved to the measurement space 1270 via the movement space 1250 depending on a first motion in which the position of the main body 1200 is changed. As another example, at least a part of the solution received in the measurement space 1270 may be moved to the first accommodation space 1210 via the movement space 1270 depending on a second motion in the position of the main body 1200 is changed, different from the first motion.

The measurement space 1270 may be provided as a space configured to receive the specimen and the solution. Specific light is radiated to the specimen and the solution received in the measurement space 1270. Concretely, a substance received in the measurement space 1270 may be measured by radiating specific light to the measurement space 1270 and detecting the intensity of reflected light. Further, the hall may be formed in at least a part of the measurement space 1270. The hall formed in the measurement space 1270 functions to assist measurement of the substance received in the measurement space 1270.

Further, the measurement space 1270 may include the absorption pad provided therein. Here, the absorption pad may include pores having a designated size. Thereby, the absorption pad may pass particles having sizes less than the designated size, and may not pass particles having sizes greater than the designated size. Consequently, among particles located in the measurement space 1270, particles having sizes less than the designated size may be moved into the absorption pad

### 2.1.3.4 Connection Relations among Respective Spaces

In the cassette according to one embodiment, the above-described plurality of spaces formed in the main body 1200, for example, the first accommodation space 1210, the second accommodation space 1230, the movement space 1250, and the measurement space 1270 may be formed to be fluidly connected.

The plurality of spaces formed in the main body 1200, for example, the first accommodation space 1210, the second accommodation space 1230, the movement space 1250, and the measurement space 1270, may be formed to be fluidly connected to one another so that, when the main body 1200 is rotated in a predetermined direction, the solutions may be moved between the respective spaces by gravity. Hereinafter, the fluidic connection relations among the respective spaces will be described in more detail with reference to the accompanying drawings.

Referring to FIG. 8, the first accommodation space 1210 and the second accommodation space 1230 may be formed to be fluidly connected to each other. Concretely, the first accommodation space 1210 and the second accommodation space 1230 may be formed to be fluidly connected to each other so that, when the position of the main body 1200 is changed, at least a part of the solution received in the second accommodation space 1230 may be moved to the first accommodation space 1210.

More concretely, the first accommodation space 1210 and the second accommodation space 1230 may be fluidly connected to each other so that, when the main body 1200 is rotated in a first direction about the z-axis in FIG. 8, the solution in the second accommodation space 1230 may be moved to the first accommodation space 1210 through the first guide G1 by gravity. In this case, the first direction may mean the x-axis direction in FIG. 8 and, when the main body 1200 is rotated by a designated angle or more in the first direction about the z-axis, the bottom surface of the second accommodation space 1230 may be higher than the bottom surface of the first accommodation space 1210 based on the y-axis, and thereby, the solution received in the second accommodation space 1230 may be moved to the first accommodation space 1210 along the first guide G1 by gravity.

Of course, even when the main body 1200 does not include the first guide G1, the solution in the second accommodation space 1230 may be moved to the first accommodation space 1210 from the second accommodation space 1230. However, when the main body 1200 includes the first guide G1, the first guide G1 has an upwardly inclined shape from one area of the bottom surface of the second accommodation space 1230 to the separation walls SW, and thus, the solution in the second accommodation space 1230 may be more easily moved to the first accommodation space 1210. That is to say, when the main body 1200 includes the first guide G1, even though the position of the main body 1200 is not much changed, the solution in the second accommodation space 120 may be moved to the first accommodation space 1210.

Further, the first accommodation space 1210 and the second accommodation space 1230 may be spatially separated from each other so that, when the main body 1200 is rotated in a direction opposite to the first direction about the z-axis in FIG. 8, the solution received in the first accommodation space 1210 is not moved to the second accommodation space 1230.

Further, the first accommodation space 1210 and the second accommodation space 1230 may be spatially separated so that, when the main body 1200 is not rotated, the solution received in the first accommodation space 1210 is moved to the second accommodation space 1230.

Further, referring to FIG. 9 to describe the relations among the first accommodation space 1210, the movement space 1250 and the measurement space 1270, the first accommodation space 1210 and the measurement space 1270 may be formed to be fluidly connected to each other via the movement space 1250.

As one example, the first accommodation space 1210 and the measurement space 1270 may be fluidly connected to each other so that at least a part of the solution received in the first accommodation space 1210 may be moved to the measurement space 1270 via the movement space 1250.

More concretely, the first accommodation space 1210 and the measurement space 1270 may be fluidly connected to each other via the movement space 1250 so that, when the main body 1200 is rotated in a second direction about the z-axis in FIG. 8, the solution received in the first accommodation space 1270 may be moved to the measurement space 1270 from the first accommodation space 1270 by gravity. In this case, the second direction may be a direction opposite to the above-described first direction. That is, the second direction may mean the x-axis direction in FIG. 8 and, when the main body 1200 is rotated by a designated angle or more in the second direction about the z-axis, the bottom surface of the first accommodation space 1210 may be higher than the bottom surface of the movement space 1250 based on the y-axis, and thereby, the solution received in the first accommodation space 1210 may be moved to the movement space 1250 by gravity.

As another example, the first accommodation space 1210 and the measurement space 1270 may be fluidly connected to each other so that at least a part of the solution received in the measurement space 1270 may be moved to the first accommodation space 1210 via the movement space 1250.

More concretely, the first accommodation space 1210 and the measurement space 1270 may be fluidly connected to each other via the movement space 1250 so that, when the main body 1200 is rotated in the first direction about the z-axis in FIG. 8, the solution in the measurement space 1270 may be moved to the first accommodation space 1270 from the measurement space 1270 by gravity.

The main body 1200 may include a plurality of guides, for example, the first guide G1, a second guide G2, and a third guide G3. The plurality of guides may be disposed in the plurality of spaces formed in the main body 1200, and may provide flow paths along which the solutions may be more smoothly moved.

Referring to FIG. 8, the second accommodation space 1230 may include the first guide G1. In this case, the first guide G1 may correspond to at least a part of the bottom surface of the second accommodation space 1230. The first guide G1 may provide a flow path along which at least a part of the solution received in the second accommodation space 1230 may be moved to the first accommodation space 1210.

More concretely, when the main body 1200 is rotated in the first direction about the z-axis in FIG. 8, the solution received in the second accommodation space 1230 may be moved to the first accommodation space 1210 from the second accommodation space 1230 through the first guide G1.

The first guide G1 may be disposed between the separation walls SW and the blocking wall BW with respect to the x-axis. Further, the first guide G1 may be disposed between at least a part of the fifth plate P5 and the first plate P1.

The first guide G1 may be formed to be inclined with respect to the bottom surface of the second accommodation space 1230. More specifically, the first guide G1 may be provided to be inclined upwardly from one area of the bottom surface of the second accommodation space 1230 to the separation walls SW. Otherwise, the first guide G1 may be provided in an upwardly inclined shape in a direction of moving the solution from the second accommodation space 1230 to the first accommodation space 1210.

Referring to FIG. 9, the movement space 1250 may include a second guide G2. At least a part of the bottom surface of the movement space 1250 may be formed as the second guide G2. The second guide G2 may provide a flow path along which at least a part of the solution received in the first accommodation space 1210 may be moved to the movement space 1270. Otherwise, the second guide G2 may provide a flow path along which at least a part of the solution received in the measurement space 1270 may be moved to the first accommodation space 1210.

Further, the movement space 1250 may include a third guide G3. At least another part of the bottom surface of the movement space 1250 may be formed as the third guide G3. The third guide G3 may provide a flow path along which at least a part of the solution received in the first accommodation space 1210 may be moved to the movement space 1270, or a flow path along which at least a part of the solution received in the measurement space 1270 may be moved to the first accommodation space 1210, in the same manner as the second guide G2.

As one example, when the main body 1200 is rotated in the second direction about the z-axis in FIG. 8, the solution received in the first accommodation space 1210 may be moved to the measurement space 1270 through the second guide G2 and the third guide G3.

As another example, when the main body 1200 is rotated in the first direction about the z-axis in FIG. 8, the solution received in the measurement space 1270 may be moved to the first accommodation space 1210 through the second guide G2 and the third guide G3.

The second guide G2 may be disposed between the first accommodation space 1210 and the third guide G3. Further, the second guide G2 may be formed to be inclined with respect to the bottom surface of the movement space 1250. Here, the direction of inclination of the second guide G2 may be opposite to the direction of inclination of the first guide G1. For example, the second guide G2 may be provided in an upwardly inclined shape from one end thereof connected to the first accommodation space 1210 to the other end thereof connected to the third guide G3.

The third guide G3 may be disposed between the second guide G2 and the measurement space 1270. Further, the third guide G3 may be formed to be inclined with respect to the bottom surface of the movement space 1250. Here, the direction of inclination of the third guide G3 may be opposite to the direction of inclination of the second guide G2. Further, the direction of inclination of the third guide G3 may be the same as the direction of inclination of the first guide G1. For example, the third guide G3 may be provided in a downwardly inclined shape from one end thereof connected to the second guide G2 to the other end thereof connected to the third guide G3.

The above-described plurality of spaces installed in the main body 1200, for example, the first accommodation space 1210, the second accommodation space 1230, the movement space 1250, and the measurement space 1270, may be fluidly connected to one another even without the above-described plurality of guides.

Concretely, the first accommodation space 1210 and the second accommodation space 1230 may be formed to be fluidly connected to each other even when the first guide G1 is not provided. Further, the first accommodation space 1210 and the measurement space 1270 may be formed to be fluidly connected to each other via the movement space 1250 even when the second guide G2 and the third guide G3 are not provided.

### 2.2 Glycated Hemoglobin Measurement Method

A method of acquiring information about an amount of glycated hemoglobin in a blood sample using the cassette according to one embodiment will be described. The method, which will be described below, may be a preferable example of the operation of the cassette according to one embodiment of the present invention. A detailed description of parts which are substantially the same as the above-described details will be omitted because it is considered to be unnecessary.

Further, although the method of acquiring information about the amount of glycated hemoglobin in the blood sample will be described below, the same technical content according to the present invention is not limited to glycated hemoglobin, and may be applied to a method of acquiring information about an albumin-to-creatinine Ratio, C-reactive protein (CRP), lysophosphatidylcholine (LPC), glycoalbumin (GA), total cholesterol (TC), and the like.

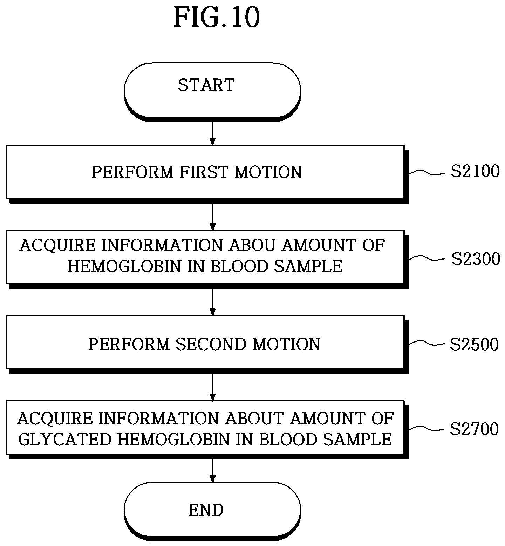

FIGs. 10 to 13 are views showing one example of a process of rotating the cassette according to one embodiment.

Referring to FIG. 10, the method of acquiring information about the amount of glycated hemoglobin in the blood sample using the cassette according to one embodiment may include performing a first motion (S2100), acquiring information about an amount of hemoglobin in the blood sample (S2400), performing a second motion (S2500), and acquiring the information about the amount of glycated hemoglobin in the blood sample (S2700).

The respective detailed configurations of the cartridge 1100 and the main body 1200 were described above, and a redundant description thereof will thus be omitted.

First, the blood sample may be loaded in the specimen collector. Here, the blood sample may be loaded in the specimen collector when the cartridge 1100 and the main body 1200 are separated from each other, as shown in FIG. 4(a). That is, when the cartridge 1100 and the main body 1200 are separated from each other, at least a part of the specimen collector 1170 may be exposed to the outside so as to receive a specimen. Here, the amount of the provided blood sample may be equal to or greater than 2 microliters, or may be equal to or greater than 5 microlitters. Otherwise, the amount of the provided blood sample may be equal to or less than 20 microlitters.

When the blood sample is loaded in the specimen collector, the blood sample may be provided to the specimen collector 1170, and the provided blood sample may be held in the specimen collector 1170.

Thereafter, as described above with reference to FIG. 4, at least a part of the cartridge 1100 may be inserted into the main body 1200. Here, external force in the negative direction of the y-axis shown in FIG. 4 may be applied to the cartridge 1100 so as to move the position of the cartridge 1100 toward the main body 1200. Otherwise, external force in the positive direction of the y-axis shown in FIG. 4 may be applied to the main body 1200. Preferably, after the main body 1200 according to one embodiment has been inserted into the measurement device, the external force may be applied. Here, the external force may be applied by a user, or may be applied by the measurement device which is separately provided.

The cartridge 1100 may be inserted into the main body 1200, after the blood sample has been loaded in the specimen collector 1170. That is, the cartridge 1100 may be inserted into main body 1200, after the blood sample has been provided to the specimen collector 1170. Thereby, the specimen collector 1170 may be inserted into the first accommodation space 1210, and the blood sample provided to the specimen collector 1170 may come into physical contact with the solution received in the first container 1110 in the first accommodation space 1210.

After the cartridge 1100 has been inserted into the main body 1200, a shaking motion may be applied to the cassette 1000 so that the blood sample and the first solution may be thoroughly mixed and glycated hemoglobin in the blood sample may react better with boronic acid combined with beads in the first solution.

After the blood sample and the first solution have been thoroughly mixed, the first motion may be performed (S2100).

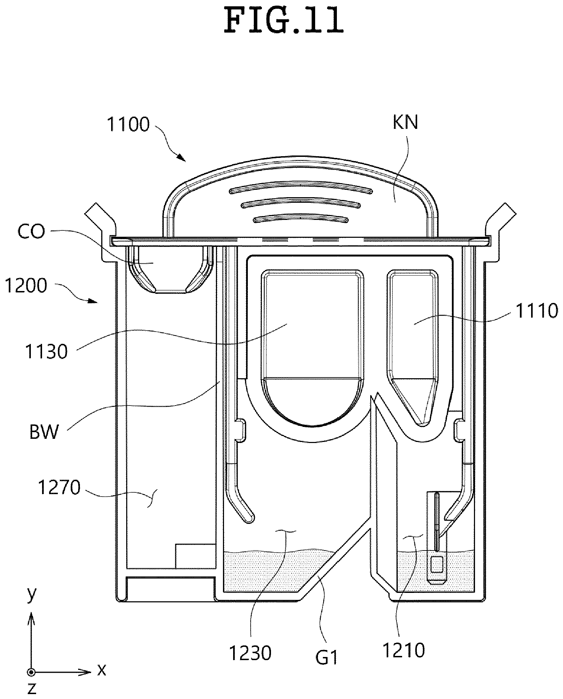

The first motion may be implemented as a motion of the main body 1200 in a first rotating direction. Here, the first rotating direction may indicate the counterclockwise direction with respect to a gaze direction seen from the positive direction of the z-axis, with reference to FIG. 11.

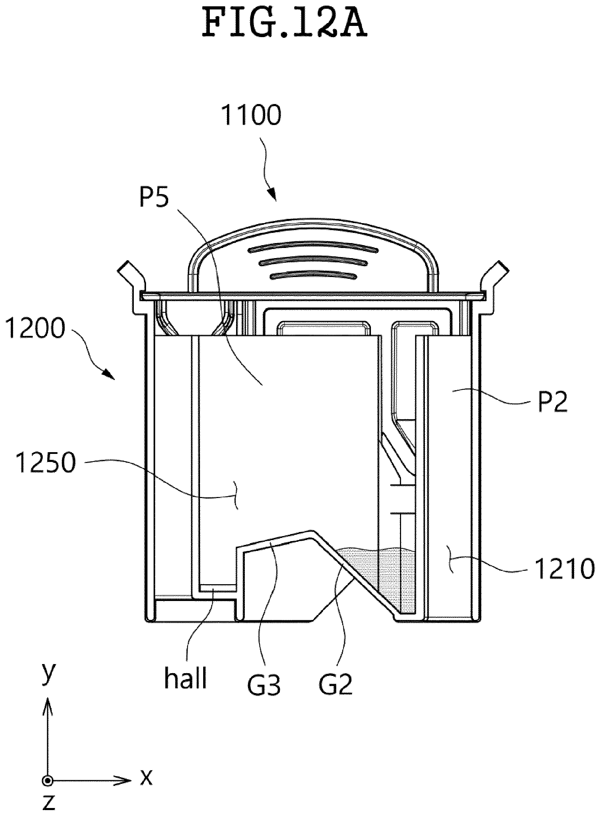

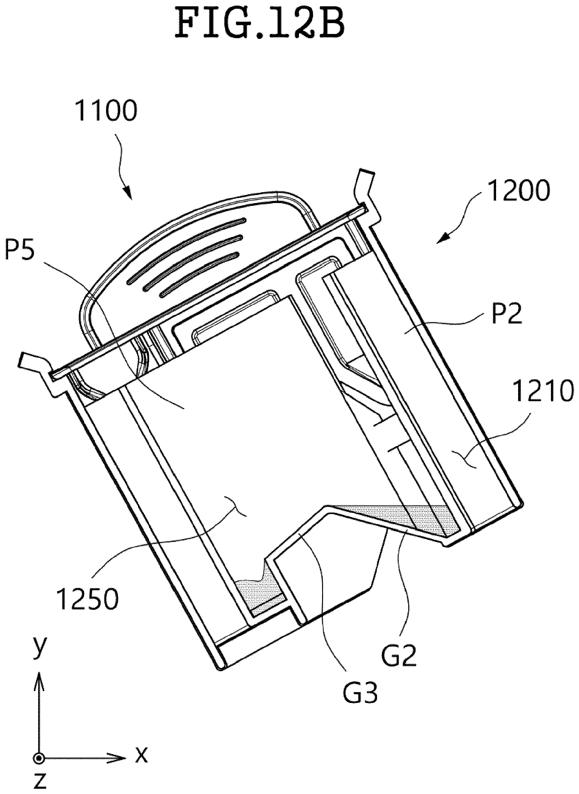

Referring to FIG. 12, the main body 1200 may have a position shown in FIG. 12(a) before performing the first motion, and may be changed to a position shown in FIG. 12(b) after performing the first motion.

As the first motion of the main body 1200 is performed, as shown in FIG. 12(b), at least a part of the solution received in the first accommodation space 1210 may be moved to the measurement space 1270 via the movement space 1250 by gravity. That is, when the main body 1200 is rotated in the first direction about the z-axis in FIG. 12, at least a part of the solution received in the first accommodation space 1210 may be moved to the measurement space 1270 from the first accommodation space 1210 by gravity.

In acquiring the information about the amount of hemoglobin in the blood sample (S2400), light of a wavelength band specifically absorbed by hemoglobin may be radiated. The information of the amount of hemoglobin moved to the measurement space 1270 may be acquired by radiating specific light to the solution and detecting the intensity of reflected light.

For example, the wavelength band specifically absorbed by hemoglobin may be a wavelength band including 415 nanometers. However, when the bio-target to be measured is albumin, the specific light should include a wavelength band specifically absorbed by albumin. For example, the wavelength band specifically absorbed by albumin may be a wavelength band including 595 nanometers.

For example, a light source configured to radiate light may be a light source provided in the above-described measurement device which is separately provided, the information about the amount of hemoglobin may be calculated depending on the amount of light sensed by a light receiver provided in the measurement device.

When the first motion has been performed, the solution including the bio-target may be received in the measurement space 1270. In this state, light in the wavelength band specifically absorbed by hemoglobin may be radiated to the surface of the solution in the measurement space 1270, and the intensity of reflected light may be acquired by detecting the reflected light by the light receiver. The intensity of the reflected light may include the information about the amount of hemoglobin.

Thereafter, the second motion may be performed (S2500).

The second motion may be implemented as a motion of the main body 1200 in a second rotating direction. Here, the second rotating direction may indicate the clockwise direction with respect to the gaze direction seen from the positive direction of the z-axis, with reference to FIG. 11.

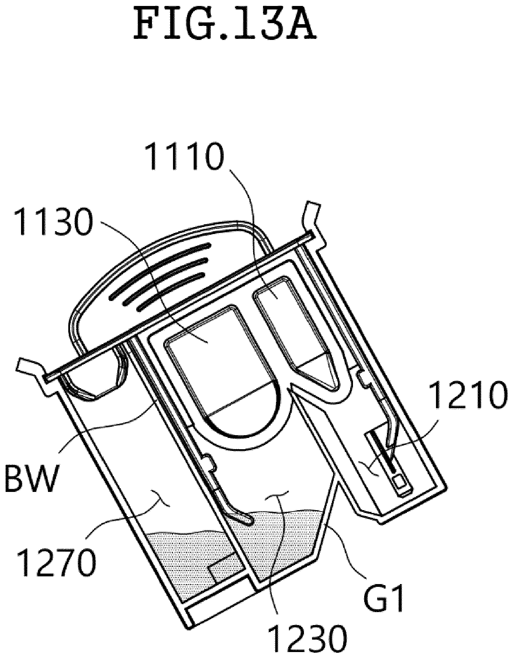

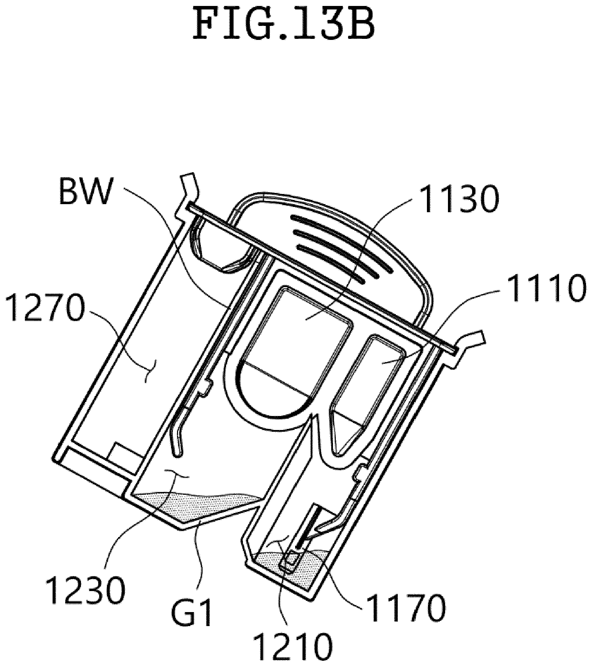

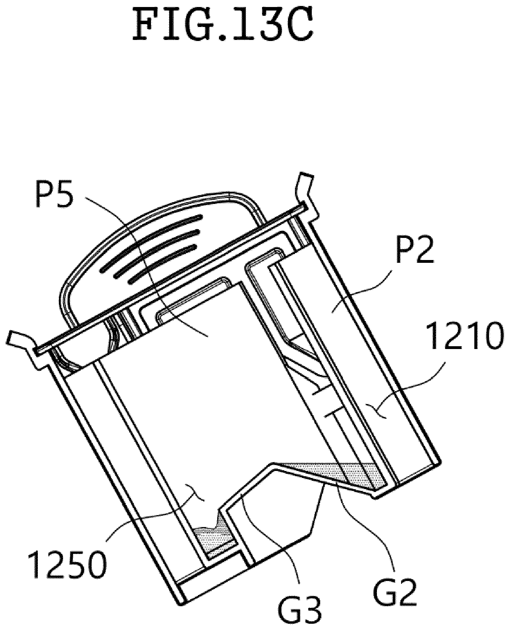

Referring to FIG. 13, the main body 1200 may have a position shown in FIG. 13(a) before performing the second motion, and may be changed to a position shown in FIG. 13(b) after performing the second motion.

As the second motion of the main body 1200 is performed, as shown in FIG. 13(b), at least a part of the solution received in the second accommodation space 1230 may be moved to the first accommodation space 1210 by gravity. That is, when the main body 1200 is rotated in the second direction about the z-axis in FIG. 13, at least a part of the solution received in the second accommodation space 1230 may be moved to the first accommodation space 1210 by gravity.

Referring to FIG. 13(c), after performing the second motion, an additional motion may be performed. The additional motion may be implemented as a motion of the main body 1200 in the first rotating direction. Here, the additional motion may correspond to the first motion described in FIG. 12, and may be performed in the same method as or in a method similar to the first motion, and a redundant description thereof will thus be omitted.

As the additional motion is performed, at least a part of the solution received in the first accommodation space 1210 may be moved to the measurement space 1270 via the movement space 1250 by gravity, as shown in FIG. 12(b) .

When this operation is performed, a mixture - the mixture including the first solution and the blood sample - located in the measurement space 1270 is washed with the second solution, and thereby, the mixture including hemoglobin is moved to the absorption pad. However, glycated hemoglobin is in a state of being coupled to beads with which boronic acid is combined, and may not thus be absorbed into the absorption pad having the pores with a size less than the beads. Consequently, in spite of washing of a third area with the second solution, glycated hemoglobin is not moved into the absorption pad. That is, after this operation, non-glycated hemoglobin is not located or only a very small amount of non-glycated hemoglobin remains in the measurement space 1270, and a very small amount of glycated hemoglobin is lost due to washing with the second solution or glycated hemoglobin is located without loss in the measurement space 1270.

In acquiring the amount of glycated hemoglobin in the blood sample (S2700), the light in the wavelength band specifically absorbed by hemoglobin may be radiated in the same manner as in acquiring the amount of hemoglobin (S2400).

In this operation, when the ratio of the intensity of reflected light to the intensity of the radiated light is measured, the information about the amount of hemoglobin located in the measurement space 1270 may be acquired. At a point in time of performing this operation, as described above, hemoglobin in the measurement space 1270 is glycated hemoglobin coupled to the beads with which boronic acid is combined, and therefore, information about the amount of glycated hemoglobin may be acquired by detecting the reflected light.

### 3. Other Embodiments - Biochemical Marker, and the Like

A cassette according to another embodiment may be a diagnostic kit configured to acquire information about a biochemical marker. For example, the biochemical marker may be at least one of microalbumin, creatinine, total cholesterol (TC), triglyceride (TG), high density lipoprotein cholesterol (HDL), aspartate aminotransferase (AST), or alanine transaminase (ALT). Otherwise, the biochemical marker may be at least one of an albumin-to-creatinine ratio, C-reactive protein (CRP), or lysophosphatidylcholine (LPC).

Since a bio-target of the biochemical marker is included in a protein, outer membranes of the protein are destroyed to perform pre-treatment, and an enzyme reaction for detection is performed. That is, the above-described first solution may include proteinase and a surfactant so as to destroy a protein structure so as to detect the target of the biochemical marker, and the second solution may include an enzyme which may specifically react with the target biochemical marker, and a dyeing material used to detect results of the enzyme reaction from the outside. As one example, the dyeing material may include peroxidase (POD) and/or a nanozyme.

The features, structures, and effects described in the above-described embodiments are included in at least one embodiment of the present invention, and are not limited to one embodiment. Further, it should be apparent to those skilled in the art that the features, structures, and effects exemplified in the respective embodiments may be combined with other embodiments or modified. Therefore, it is to be understood that these combinations and modifications fall within the scope of the invention.

Further, while the present invention has been explained in relation to its embodiments, the embodiments are only examples and are not intended to limit the present invention, and it is to be understood that various modifications and applications thereof within the spirit and scope of the present disclosure will become apparent to those skilled in the art upon reading the specification. That is, it is to be understood that the respective elements described in detail in the embodiments may be modified. Further, it is to be understood that differences related to these modifications and applications are interpreted to fall within the scope and spirit of the present invention as disclosed in the accompanying claims.

## Claims

1. A cassette for measuring a reaction between a specimen and a solution, having an x-axis, a y-axis, and a z-axis configured to be orthogonal to one another, the cassette comprising:
a cartridge comprising a first container and a second container; and
a main body configured such that the first container and
the second container are capable of being inserted thereinto,
wherein the main body comprises:
a first plate and a second plate disposed in an x-axis direction and located at different positions on the z-axis;
a third plate and a fourth plate disposed in a z-axis direction and located at different positions on the x-axis;
a fifth plate disposed in the x-axis direction between at least a part of the second plate and the first plate;
a blocking wall disposed in the z-axis direction between the third plate and the fourth plate and disposed between the first plate and the fifth plate with respect to the z-axis; and
separation walls disposed between the first plate and the fifth plate with respect to the z-axis, between the third plate and the blocking wall, wherein:
a first accommodation space is defined by the first plate, the second plate, the third plate and the separation walls;
a second accommodation space is defined by the first plate, the fifth plate, the blocking wall and the separation walls;
a measurement space is defined by the first plate, the second plate, the fourth plate, the fifth plate and the blocking wall;
a movement space is defined by the second plate and the fifth plate;
the movement space is fluidly connected to the first accommodation space and the measurement space;
the first accommodation space and the second accommodation space are fluidly connected to each other so that, when the main body is rotated in a first direction about the z-axis, a solution received in the second accommodation space is moved to the first accommodation space from the second accommodation space by gravity; and
the first accommodation space and the measurement space are fluidly connected to each other via the movement space so that, when the main body is rotated in a second direction opposite to the first direction about the z-axis, a solution received in the first accommodation space is moved to the measurement space from the first accommodation space by gravity.

2. The cassette according to claim 1, wherein the separation walls comprise a first separation wall and a second separation wall,
wherein the first separation wall is connected to the first plate and is disposed in the z-axis direction between the third plate and the blocking wall, and the second separation wall is connected to the first separation wall and the fifth plate.

3. The cassette according to claim 1, wherein:
a first container is configured to receive a first solution, and a second container is configured to receive a second solution; and
when at least a part of the cartridge is inserted into the main body, the first solution is received in the first accommodation space, and the second solution is received in the second accommodation space.

4. The cassette according to claim 3, wherein:
the cartridge further comprises a specimen collector configured to hold the specimen;
when at least the part of the cartridge is inserted into the main body, the specimen and the first solution are mixed in the first accommodation space.

5. The cassette according to claim 4, wherein, when the main body is rotated in the second direction about the z-axis, the first solution mixed with the specimen received in the first accommodation space is moved to the measurement space from the first accommodation space via the movement space by gravity.

6. The cassette according to claim 5, wherein, when the main body is rotated in the second direction about the z-axis, at least a part of the first solution mixed with the specimen and the second solution received in the first accommodation space are moved to the measurement space from the first accommodation space via the movement space by gravity.

7. The cassette according to claim 1, wherein, when the main body is rotated in the first direction about the z-axis, the solution received received in the second accommodation space is moved to the first accommodation space from the second accommodation space through a first guide; and
the first guide is provided such that at least a part thereof is disposed between the separation walls and the blocking wall, and has an upwardly inclined shape from one area of a bottom surface of the second accommodation space to the separation walls.

8. The cassette according to claim 1, wherein, when the main body is rotated in the second direction about the z-axis, the solution received in the first accommodation space is moved to the measurement space from the first accommodation space through a second guide configured to form at least a part of a bottom surface of the movement space.

9. The cassette according to claim 8, wherein:
the second guide comprises a first detailed guide and a second detailed guide;
the first detailed guide is disposed between the first accommodation space and the second detailed guide;
the second detailed guide is disposed between the first detailed guide and the measurement space;
the first detailed guide has an upwardly inclined shape from one end thereof connected to the first accommodation space to a remaining end thereof connected to the second detailed guide; and
the second detailed guide has a downwardly inclined shape from one end thereof connected to the first detailed guide to a remaining end thereof.

10. The cassette according to claim 1, wherein a first area of the fifth plate located between the blocking wall and the fourth plate with respect to the x-axis forms at least a part of one side surface of the measurement space; and
a hall configured such that the solution passes therethrough is formed at a lower end of the first area.

11. The cassette according to claim 1, wherein a bottom surface of the first accommodation space and a bottom surface of the second accommodation space do not come into contact with each other, and are physically separated from each other.

12. The cassette according to claim 1, wherein the second plate comprises a first plane, a second plane, a third plane, a fourth plane, and a fifth plane, wherein:
the first plane, the third plane, and the fifth plane are disposed in the x-axis direction, and a distance from the first plate to the first plane and the fifth plane in the z-axis direction is less than a distance from the first plate to the third plane in the z-axis direction; and
the second plane comes into contact with the first plane and the third plane, and the fourth plane comes into contact with the third plane and the fifth plane.

13. The cassette according to claim 2, wherein the cartridge further comprises a first container cover configured to seal the first container, a second container cover configured to seal the second container, and a holder, wherein:
when at least a part of the cartridge is inserted into the main body, a position of the holder with respect to the cartridge is changed due to contact with the second plate or the fifth plate; and
when the position of the holder is changed, at least a part of the first container cover and at least a part of the second container cover are respectively separated from the first container and the second container by the holder.
